# EUROPEAN PATENT SPECIFICATION

(11) **EP 4 014 045 B1**
(45) Date of publication and mention of the grant of the patent: **29.11.2023**
(21) Application number: 20753373.8
(22) Date of filing: 12.08.2020
(51) Int. Cl.: G01N 33/574, G01N 33/577, G01N 33/68

(54) **DIAGNOSIS OF CANCER USING DETECTION OF AUTO-ANTIBODIES DIRECTED AGAINST PD1 AND PD-L1**
KREBSDIAGNOSE MIT AUTO-ANTIKÖRPERN GEGEN PD1 UND PD-L1
DIAGNOSTIC DE CANCER AVEC D'AUTO-ANTICORPS CONTRE PD1 ET PD-L1

(30) Priority: 13.08.2019 EP 19191445
(43) Date of publication of application: 22.06.2022
(73) Proprietor: CellTrend GmbH, 14943 Luckenwalde (DE)
(72) Inventor: HEIDECKE, Harald, 14169 Berlin (DE); SCHULZE-FORSTER, Kai, 12207 Berlin (DE)
(74) Representative: Vossius & Partner Patentanwälte Rechtsanwälte mbB
(86) International application number: PCT/EP2020/072614
(87) International publication number: WO 2021/028468

(56) References cited:
- WO-A1-2015/117951
- WO-A1-2016/061064
- WO-A1-2018/234577
- QIAOYUN TAN ET AL: "Autoantibody profiling identifies predictive biomarkers of response to anti-PD1 therapy in cancer patients", THERANOSTICS, vol. 10, no. 14, 1 January 2020 (2020-01-01), pages 6399-6410, XP055729571, AU ISSN: 1838-7640, DOI: 10.7150/thno.45816
- ISABEL K MACDONALD ET AL: "Autoantibodies: Opportunities for Early Cancer Detection", TRENDS IN CANCER, vol. 3, no. 3, 31 March 2017 (2017-03-31), pages 198-213, XP008185827, ISSN: 2405-8033, DOI: 10.1016/J.TRECAN.2017.02.003 [retrieved on 2017-03-05]
- HUI SHI ET AL: "Elevated serum autoantibodies against co-inhibitory PD-1 facilitate T cell proliferation and correlate with disease activity in new-onset systemic lupus erythematosus patients", ARTHRITIS RESEARCH & THERAPY, vol. 19, no. 1, 9 March 2017 (2017-03-09), XP055729585, DOI: 10.1186/s13075-017-1258-4
- DOROTHEA SONJA SCHOTT ET AL: "Sensitive detection of PD-L1 expression on circulating epithelial tumor cells (CETCs) could be a potential biomarker to select patients for treatment with PD-1/PD-L1 inhibitors in early and metastatic solid tumors", ONCOTARGET, vol. 8, no. 42, 22 September 2017 (2017-09-22), pages 72755-72772, XP055729640, DOI: 10.18632/oncotarget.20346
- DAQIAN GU ET AL: "Soluble immune checkpoints in cancer: production, function and biological significance", JOURNAL FOR IMMUNOTHERAPY OF CANCER, BIOMED CENTRAL LTD, LONDON, UK, vol. 6, no. 1, 27 November 2018 (2018-11-27), pages 1-14, XP021263345, DOI: 10.1186/S40425-018-0449-0
- KATERINA ANCEVSKI HUNTER ET AL: "PD-L1 Testing in Guiding Patient Selection for PD-1/PD-L1 Inhibitor Therapy in Lung Cancer", MOLECULAR DIAGNOSIS AND THERAPY, vol. 22, no. 1, 8 November 2017 (2017-11-08), pages 1-10, XP055696977, NZ ISSN: 1177-1062, DOI: 10.1007/s40291-017-0308-6

## Description

### Field of the invention

The present invention is in the field of diagnostic and prognostic methods for cancer. It relates to a method for the diagnosis, prognosis, risk assessment, risk stratification, monitoring, therapy guidance and/or therapy control of cancer in a subject comprising the determination of the level of an anti-PD1 auto-antibody and/or an anti-PD-L1 auto-antibody in a blood sample of said subject.

### Background of the invention

Tumor cells evade immunosurveillance and progress through different mechanisms, including activation of so-called immune checkpoint pathways that suppress antitumor immune responses (Darvin et al., Experimental & Molecular Medicine (2018) 50:165). These immune checkpoints are mostly represented by T-cell receptor binding to ligands on cells in the surrounding microenvironment, forming immunological synapses which then regulate the functions of the T cell, which become specialized, or "polarized", to perform different activities (Oiseth et al., J Cancer Metastasis Treat (2017) 3:250-261). T cells recognize and become activated against peptide antigens through ligation of T cell surface receptors. Two signals are required for T cell activation. The first signal is generated by the binding of major histocompatibility complex (MHC)-presented immunogenic peptide antigen to the heterodimeric T cell receptor (TCR). The second signal, also referred to as co-stimulation, is transduced via ligation of the T cell costimulatory surface receptor CD28 to its ligand CD80 (also known as B7-1) or CD86 (also known as B7-2) on the surface of professional antigen-presenting cells (APCs). Once activated, T cells begin to express co-inhibitory cell surface receptors, such as cytotoxic T lymphocyte antigen 4 (CTLA4) and programmed cell death 1 (PD1). Like CD28, CTLA4 binds CD80 and CD86, but with significantly higher affinity. CTLA4 ligation with CD80 or CD86 blocks co-stimulation (second signal) and prevents continued T cell activation. Blockade of the CTLA4-CD80 or CTLA4-CD86 interaction therefore promotes activation of T cells in secondary lymphoid organs. Binding of PD1 to its ligand, PD1 ligand 1 (PD-L1), inhibits signaling downstream of the TCR, thereby blocking the first signal. PD-L1 is frequently expressed on tumors or in the tumor microenvironment (Havel et al., Nat Rev Cancer (2019) 19:133-150).

Macdonald et al. (2017) (Trends in Cancer 3(3):198-213) teaches that many tumor antigens are proteins that are overexpressed during the course of the disease (cancer or autoimmunodiseases) and that the corresponding autoantibodies can be found in serum.

WO 2018/234577 A1 relates to detecting tumor-associated antigens and auto-antibodies against said tumor-associated antigens in samples of prostate cancer patients.

WO 2015/117951 A1 discloses a method for diagnosis of cancer comprising determining the level of antibodies against epidermal growth factor receptor (EGFR) in a sample from a subject to be diagnosed.

Shi et al. (2017) (Arthritis Research & Therapy 19(1):1-15) teaches elevated anti-PD1 autoantibody levels in systemic lupus erythematosus patients.

Schott et al. (2017) (Oncotarget 8(42):72755-72772) and WO 2016/061064 A1 disclose using the level of expression of PD-L1 in circulating tumor cells obtained from blood as biomarker for monitoring and predicting cancer therapy with anti-PD1/PD-L1 antibodies.

Gu et al. (2018) (Journal for Immunotherapy of Cancer 6(1):1-14) discloses using the level of soluble immune checkpoint molecules such as PD-1 and PD-L1 in plasma as biomarker for cancer diagnosis and prognosis of cancer therapy with anti-PD1/PD-L1 antibodies.

Hunter et al. (2018) (Molecular Diagnosis and Therapy 22(1):1-10) discloses using the level of PD-L1 in tumor tissues or tumor infiltrating cells as biomarker for predicting the likelihood to response to a therapy with anti-PD1/anti-PD-L1 antibodies.

Monoclonal antibodies (Mabs) against CTLA4, PD1 and PD-L1 are known as "immune checkpoint inhibitors" (ICIs). They are important therapeutic options because they are much less toxic than conventional cancer therapies, are easier to prepare and administer than other types of cancer immunotherapeutics and have great potential for widespread application.

The humanized anti-CTLA4 antibody ipilimumab has doubled 10-year survival for metastatic melanoma compared with historical data and was approved by the United States Food and Drug Administration (FDA) for clinical use in 2011. Blockade of another immune checkpoint molecule, PD1 or its ligand PD-L1, was shown to provide a survival advantage in a number of different malignancies, with higher response rates and lower incidence of side effects than anti-CTLA4. Accordingly, antibodies targeting the PD1-PDL1 axis have been approved as secondline or first- line therapies for an ever-growing list of malignancies, including melanoma, lymphoma, lung cancers, renal cell cancer (RCC), head and neck squamous cell cancer (HNSCC), bladder cancer, liver cancer and gastroesophageal cancer. However, despite these substantial advancements in clinical care, the majority of patients receiving ICIs do not derive benefit. Whereas anti-CTLA-4 antibodies (ipilimumab and tremelimumab), anti-PD-1 antibodies (nivolumab and pembrolizumab), and anti-PD-L1 antibodies (atezolizumab, avelumab and durvalumab) have produced remarkable results regarding tumor control in many malignancies, response is often followed by relapse and disease progression.

Therefore, there exists intense interest in identifying and developing predictive biomarkers of ICI response and for monitoring ICI treatment response to enable a precision medicine approach in cancer immunotherapy. There is a need for effective biomarker-based patient selection and monitoring.

### Summary of the invention

The inventors now for the first time found auto-antibodies against PD-1 and PD-L1 in blood samples from cancer patients. They found that the levels of auto-antibodies against PD1 and PD-L1 are increased in these patients as compared to healthy subjects and are dependent on tumor load. Hence, the subject of the present invention is a method for the diagnosis, prognosis, risk assessment, risk stratification, monitoring, therapy guidance and/or therapy control of cancer in a subject comprising the determination of the level of an anti-PD1 auto-antibody and/or an anti-PD-L1 auto-antibody in a blood sample of said subject. In the method of the invention, preferably only the auto-antibodies against PD1 or PD-L1, respectively, are detected to avoid a potential masking effect by the therapeutic antibody. In other words, it is advantageous that the level of an anti-PD1 antibody is determined in a sample of a subject, wherein the subject does not currently receive treatment with an anti-PD1 antibody, or the level of an anti-PD-L1 antibody is determined in a sample of the subject, wherein the subject does not currently receive treatment with an anti-PD-L1 antibody.

The level of the autoantibody can be compared to a control level, e.g. the control level is derived from a sample of a healthy individual or samples from a group of healthy individuals. Other controls can for example be based on tumor-free individuals or patients with relapse or Known responders or non-responders for a certain therapy depending in the particular aspect to be diagnosed. However, it is also a typical aspect of the present invention that the level of the anti-PD1 or anti-PD-L1 antibodies are compared over time in the same subject; in particular, samples can be taken, and the level of the antibodies can be determined at different points of time in the same individuals, e.g. before and during treatment. This allows a monitoring of the effect of the cancer treatment, typically with immune checkpoint inhibitors. Typically, the higher levels of the antibodies in the subject's sample the higher the tumor load is.

The sample herein is a blood sample, such as whole blood, serum or plasma, preferably serum.

The cancer can in particular be a solid tumor, more in particular the cancer can be selected from the group consisting of lung cancer (e.g. the lung cancer is small cell lung cancer or non-small-cell lung carcinoma, preferably extensive stage small cell lung cancer), bladder cancer, breast cancer (e.g. advanced triple-negative breast cancer), colorectal cancer, melanoma, renal cell carcinoma (RCC), pancreatic cancer, gastric cancer, liver cancer, gastroesophageal cancer, lymphoma, head and neck squamous cell carcinoma (HNSCC) and ovarian cancer.

### Description of the Drawings

**Fig. 1****:** Anti-PD1 and anti-PD-L1 antibody levels in serum samples of healthy donors (HD; n = 4), HNSCC patients with tumor before the beginning of atezolizumab treatment (n = 5) and tumor-free HNSCC patients after atezolizumab treatment (n = 4).
**Fig. 2****:** Anti-PD1 and anti-PD-L1 antibody levels in serum samples of three different individual HNSCC patients (#1 to #3) with tumor before the beginning of atezolizumab treatment and after atezolizumab treatment.

### Detailed Description of the Invention

The present invention is based on the surprising finding of the inventors that the levels of autoantibodies against PD1 and PD-L1 are increased in blood samples (e.g. serum) of cancer patients as compared to healthy subjects. Moreover, they found that the levels of these antibodies are dependent on the tumor status of said patients. For example, it was found that patients that are tumor-free after treatment have lower auto-antibody titres than patients before treatment or at the beginning of the treatment. Auto-antibodies directed against PD1 and PD-L1, respectively, were not known until today. The present inventors for the first time demonstrate the presence of such antibodies as well as their diagnostic and predictive value.

Thus, the subject of the present invention is a method for the diagnosis, prognosis, risk assessment, risk stratification, monitoring, therapy guidance and/or therapy control of cancer in a subject comprising the determination of the level of an anti-PD1 antibody and/or an anti-PD-L1 antibody in a sample of a bodily fluid of said subject. The invention aims at the detection of auto-antibodies in the samples of said subjects which are preferably cancer patients. Hence, it is preferred in the context of the present invention that in case the subject is presently treated with a therapeutic anti-PD1 antibody that anti-PD-L1 auto-antibodies are detected for the diagnosis or prognosis. Similarly, it is preferred in the context of the present invention that in case the subject is presently treated with a therapeutic anti-PD-L1 antibody that anti-PD1 autoantibodies are detected for the diagnosis or prognosis. However, this is not an absolute requirement as the level of therapeutic antibodies in the circulation of the patients can be predetermined or estimated based on experience and parameters such as plasma half-life or elimination rates, and therefore the level of auto-antibodies can be calculated from the raw data in such situations.

The method of the invention can be used for diagnosing or monitoring the tumor status or volume of said subject. For example, the method can be used to assess whether a subject is tumor-free. In another aspect, the method can be used to assess whether the subject is a responder or non-responder to a particular treatment. The method can also be used to guide treatment, e.g. indicate when further treatment is necessary. It can also be used to detect tumor relapse in the subject.

"PD1" and "PD-L1" refer to the "programmed cell death 1" cell surface receptor and its ligand receptor "PD1 ligand 1", respectively. Sequences and properties of human can be derived from the UniProtKB database entry Q15116 (PDCD1_HUMAN) (https://www.uniprot.org/uniprot/Q15116) and NCBI gene ID 5133 (https://www.ncbi.nlm.nih.gov/gene/5133). Sequences and properties of human PD-L1 can be derived from the UniProtKB database entry Q9NZQ7 (PD1L1_HUMAN) (https://www.uniprot.org/uniprot/Q9NZQ7) and NCBI gene ID 29126 (https://www.ncbi.nlm.nih.gov/gene/29126).

"Cancer" in connection with the present invention is to be understood as any diseases involving unregulated cell growth. Cancer in this regard is a disease where cells divide and grow uncontrollably resulting in the formation of malignant tumors. In a preferred aspect of the present invention "cancer" refers to a cancer which is associated with PD-L1 expression on the surface of the cancer cells. However, while PD-L1 expressing tumors are known to be more likely to be susceptible to treatment with checkpoint inhibitors, there are for example patients with PD-L1 negative tumors who also show response to anti-PD1 treatment. It is preferred that the cancer in the context of the present invention is a cancer that is susceptible to checkpoint inhibitor treatment and in particular to anti-PD1 and/or anti-PD-L1 therapy. The cancer can in particular be a solid tumor, more in particular the cancer can be selected from the group consisting of lung cancer (e.g. the lung cancer can in particular be small cell lung cancer or nonsmall-cell lung carcinoma, preferably extensive stage small cell lung cancer), bladder cancer, breast cancer (e.g. advanced triple-negative breast cancer), colorectal cancer, melanoma, renal cell carcinoma (RCC), pancreatic cancer, gastric cancer, liver cancer, gastroesophageal cancer, lymphoma, head and neck squamous cell carcinoma (HNSCC) and ovarian cancer. In a particular aspect, the cancer is HNSCC.

Hence, in one very particular aspect, the present invention relates to a method for the diagnosis, prognosis, risk assessment, risk stratification, monitoring, therapy guidance and/or therapy control of head and neck squamous cell carcinoma in a subject comprising the determination of the level of an anti-PD1 antibody and/or an anti-PD-L1 antibody in a blood, serum or plasma sample of said subject. Even more in particular, the subject is a human HNSCC patient undergoing treatment with atezolizumab and the method is used to monitor, guide or control the treatment.

Further parameters and markers can also be considered in addition to diagnose the subject. In the context of the present invention the subject to be diagnosed is a mammal, preferably a human. The subject is preferably a human suspected to have cancer or more typically a subject that has been diagnosed with cancer and is undergoing cancer therapy.

According to the invention, the sample is a blood sample, a serum sample, or a plasma sample, preferably a serum sample or a plasma sample. Serum samples are particularly preferred samples in the context of the present invention.

Samples may be subjected to one or more pre-treatments prior to use in the present invention. Such pre-treatments include, but are not limited to dilution, filtration, centrifugation, concentration, sedimentation, precipitation, and dialysis. Pre-treatments may also include the addition of chemical or biochemical substances to the solution, such as acids, bases, buffers, salts, solvents, reactive dyes, detergents, emulsifiers, chelators.

"Plasma" in the context of the present invention is the virtually cell-free supernatant of blood containing anticoagulant obtained after centrifugation. Exemplary anticoagulants include calcium ion binding compounds such as EDTA or citrate and thrombin inhibitors such as heparinates or hirudin. Cell-free plasma can be obtained by centrifugation of the anticoagulated blood (e.g. citrated, EDTA or heparinized blood) for at least 15 minutes at 2000 to 3000 g.

"Serum" is the liquid fraction of whole blood that is collected after the blood is allowed to clot. When coagulated blood (clotted blood) is centrifuged serum can be obtained as supernatant. It does not contain fibrinogen, although some clotting factors remain.

In a further embodiment the methods according to the present invention may further comprise an initial step of providing the sample, e.g. of blood, plasma or serum, of a subject.

In the method of the present invention, the anti-PD1 or anti-PD-L1 antibody is preferably detected in an immunoassay. Suitable immunoassays may be selected from the group of immunoprecipitation, enzyme immunoassay (EIA)), enzyme-linked immunosorbenassys (ELISA), radioimmunoassay (RIA), fluorescent immunoassay, a chemiluminescent assay, an agglutination assay, nephelometric assay, turbidimetric assay, a Western Blot, a competitive immunoassay, a noncompetitive immunoassay, a homogeneous immunoassay a heterogeneous immunoassay, a bioassay and a reporter assay such as a luciferase assay or Luminex ^{®} Assays. Preferably herein the immunoassay is an enzyme linked immunosorbent assay (ELISA).

The immunoassays can be homogenous or heterogeneous assays, competitive and noncompetitive assays. In a particularly preferred embodiment, the assay is in the form of a sandwich assay, which is a non-competitive immunoassay, wherein the anti-PD1 or anti-PD-L1 antibody (i.e. the "analyte") to be detected and/or quantified is allowed to bind to an immobilized PD1 or PD-L1 protein, respectively, or immunogenic peptide fragment thereof and to a secondary antibody. The PD1 or PD-L1 protein, respectively, or fragment thereof (i.e. a peptide), may e.g., be bound to a solid phase, e.g. a bead, a surface of a well or other container, a chip or a strip, and the secondary antibody is an antibody which is labeled, e.g. with a dye, with a radioisotope, or a reactive or catalytically active moiety such as a peroxidase, e.g. horseradish peroxidase. The amount of labeled antibody bound to the analyte is then measured by an appropriate method. The general composition and procedures involved with "sandwich assays" are well-established and known to the skilled person (The Immunoassay Handbook, Ed. David Wild, Elsevier LTD, Oxford; 3rd ed. (May 2005), ISBN-13: 978-0080445267*;* Hultschig C et al., Curr Opin Chem Biol. 2006 Feb;10(1):4-10. PMID: 16376134*).* Sandwich immunoassays can for example be designed as one-step assays or as two-step assays.

The detectable label may for example be based on fluorescence or chemiluminescence. The labelling system comprises rare earth cryptates or rare earth chelates in combination with a fluorescence dye or chemiluminescence dye, in particular a dye of the cyanine type. In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, such as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

In the context of the present invention, chemiluminescence based assays comprise the use of dyes, based on the physical principles described for chemiluminescent materials in Kirk-Othmer, Encyclopedia of chemical technology, 4th ed., executive editor, J. I. Kroschwitz; editor, M. Howe-Grant, John Wiley & Sons, 1993, vol.15, p. 518-562, including citations on pages 551-562. Preferred chemiluminescent dyes are acridiniumesters.

The "sensitivity" of an assay relates to the proportion of actual positives which are correctly identified as such, i.e. the ability to identify positive results (true positives positive results / number of positives). Hence, the lower the concentrations of the analyte that can be detected with an assay, the more sensitive the immunoassay is. The "specificity" of an assay relates to the proportion of negatives which are correctly identified as such, i.e. the ability to identify negative results (true negatives / negative results). For an antibody the "specificity" is defined as the ability of an individual antigen binding site to react with only one antigenic epitope. The binding behaviour of an antibody can also be characterized in terms of its "affinity" and its "avidity". The "affinity" of an antibody is a measure for the strength of the reaction between a single antigenic epitope and a single antigen binding site. The "avidity" of an antibody is a measure for the overall strength of binding between an antigen with many epitopes and multivalent antibodies.

An "immunogenic peptide" or "antigenic peptide" as used herein is a portion of the PD1 or PD-L1 protein, respectively, that is recognized (i.e., specifically bound) by the anti-PD1 or anti-PD-L1 antibody, respectively. Such immunogenic peptides generally comprise at least 5 amino acid residues, more preferably at least 10, and still more preferably at least 20 amino acid residues of PD1 or PD-L1, respectively. However, they may also comprise at least 30, 40, 50, 60, 70, 80, 90, 100, 110, 120, 130, 140 or 150 amino acid residues.

For the purposes of the immunoassays that can be used in the context of the methods of the invention, PD1 or PD-L1 can be produced by expression in cells, preferably eukaryotic cells or in cell free, preferably eukaryotic cell free systems. Hence, in the assays and methods of the invention PD1 or PD-L1 may be present in its natural cellular environment and can be used together with the material associated with the receptor in its natural state as well as in isolated form. Suitable expression systems include Chinese hamster ovary (CHO) cells overexpressing the human PD1 or PD-L1 proteins. Hence, cell extracts (particularly extracts from CHO cells overexpressing the human PD1 or PD-L1 proteins) can be used to detect anti- PD1 or anti-PD-L1 antibodies. Based on the weight of the whole receptor in the preparation (e.g. the "extract") to be used according to the invention, the isolated receptor should account for at least 0.5%, preferably at least 5% more preferably at least 25%, and in a particular preferred embodiment at least 50%. The receptor is preferably used in isolated form, i.e. essentially free of other proteins, lipids, carbohydrates or other substances naturally associated with the receptor. "Essentially free of" means that the receptor is at least 75%, preferably at least 85%, more preferably at least 95% and especially preferably at least 99% free of other proteins, lipids, carbohydrates or other substances naturally associated with the receptor.

In particular, the method of the present invention comprises the steps of
(a) contacting the sample with PD1 or PD-L1 or an antigenic peptide fragment under conditions allowing for the formation of a complex between anti-PD1 or anti-PD-L1 antibodies with PD1 or PD-L1 or the antigenic peptide fragment thereof,
(b) detecting the complex.

PD1 or PD-L1 or the antigenic peptide fragment thereof may preferably be immobilized on a surface. The complex may for example be detected using a secondary antibody against the Fc portion of the anti-PD1 or anti-PD-L1 antibody. When the anti-PD1 or anti-PD-L1 antibody is an IgG-antibody, the secondary antibody may be an anti-IgG antibody from another species (e.g. goat anti-human-IgG). The secondary antibody may for example be labeled with a detectable marker, e.g. a peroxidase.

In the context of the present invention, the levels of the anti-PD1 or anti-PD-L1 antibodies a may be analyzed in a number of fashions well known to a person skilled in the art. For example, each assay result obtained may be compared to a "normal" value, or a value indicating a particular disease state or outcome (e.g. treatment response, tumor status, tumor volume). A particular, diagnosis/prognosis may depend upon the comparison of each assay result to such a value, which may be referred to as a diagnostic or prognostic "threshold". In certain embodiments, assays for one or more diagnostic or prognostic indicators are correlated to a condition or disease by merely the presence or absence of the indicator(s) in the assay. For example, an assay can be designed so that a positive signal only occurs above a particular threshold concentration of interest, and below which concentration the assay provides no signal above background.

The sensitivity and specificity of a diagnostic and/or prognostic test depends on more than just the analytical "quality" of the test, they also depend on the definition of what constitutes an abnormal result. In practice, Receiver Operating Characteristic curves (ROC curves), are typically calculated by plotting the value of a variable versus its relative frequency in "normal" (e.g. apparently healthy individuals not having cancer) and "disease" populations. Likewise, other states of the disease or treatment can be compared (e.g. response to treatment, tumor status). For any particular marker, a distribution of marker levels for subjects with and without a disease (or specific disease state) will likely overlap. Under such conditions, a test does not absolutely distinguish normal from disease with 100% accuracy, and the area of overlap indicates where the test cannot distinguish normal from disease. A threshold is selected, below which the test is considered to be abnormal and above which the test is considered to be normal. The area under the ROC curve is a measure of the probability that the perceived measurement will allow correct identification of a condition. ROC curves can be used even when test results don't necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art. See, *e.g.,* Hanley et al. 1982. Radiology 143: 29-36*.* Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5% of a given measurement.

The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

In other embodiments, a positive likelihood ratio, negative likelihood ratio, odds ratio, or hazard ratio is used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group.

The skilled artisan will understand that associating a diagnostic or prognostic indicator, with a diagnosis or with a prognostic risk of a future clinical outcome is a statistical analysis. For example, a marker level of lower than X may signal that a patient is more likely to suffer from an adverse outcome than patients with a level more than or equal to X, as determined by a level of statistical significance. Additionally, a change in marker concentration from baseline levels may be reflective of patient prognosis, and the degree of change in marker level may be related to the severity of adverse events. Statistical significance is often determined by comparing two or more populations, and determining a confidence interval and/or a p value. See, *e.g.,* Dowdy and Wearden, Statistics for Research, John Wiley & Sons, New York, 1983*.* Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001.

Suitable threshold levels for the stratification of subjects into different groups (categories) have to be determined for each particular combination of auto-antibodies, disease and/or medication. This can e.g. be done by grouping a reference population of patients according to their level of the respective auto-antibodies into certain quantiles, e.g. quartiles, quintiles or even according to suitable percentiles. For each of the quantiles or groups above and below certain percentiles, hazard ratios can be calculated comparing the risk for an adverse outcome, i.e. an "cancer" or a "non response", e.g. in terms of survival rate/mortality, between those patients who have received a certain medication and those who did not, or in terms of presence and absence of cancer in patients. In such a scenario, a hazard ratio (HR) above 1 indicates a higher risk for an adverse outcome for the patients who have received a treatment than for patients who did not. A HR below 1 indicates beneficial effects of a certain treatment in the group of patients. A HR around 1 (e.g. +/- 0.1) indicates no elevated risk but also no benefit from medication for the particular group of patients. By comparison of the HR between certain quantiles of patients with each other and with the HR of the overall population of patients, it is possible to identify those quantiles of patients who have an elevated risk and those who benefit from medication and thereby stratify subjects according to the present invention.

In some cases, presence of cancer, relapse and/or mortality upon treatment will affect patients with high levels (e.g. in the fifth quintile) of anti-PD1 or anti-PD-L1 antibodies. However, with the above explanations, a skilled person is able to identify those groups of patients having cancer, those groups that do respond to a medication and those groups that do not respond to the medication. In another embodiment of the invention, the diagnosis, risk for relapse of cancer and/or mortality and/or outcome for a patient are determined by relating the patient's individual level of auto-antibody to certain percentiles (e.g. 97.5^{th} percentile) of a healthy population.

Kaplan-Meier estimators may be used for the assessment or prediction of the outcome or risk (e.g. diagnosis, relapse, progression or morbidity) of a patient.

The treatment that can be assessed and monitored with the method of the present invention can be a treatment with one or more immune checkpoint inhibitors, i.e. with an anti-PD1, an ani-PD-L1 and/or an anti-CTLA4 antibody. The immune-checkpoint inhibitor herein may, thus, be selected from the group consisting of atezolizumab, avelumab, durvalumab, nivolumab, pembrolizumab, and cemiplimab and ipilimumab, preferably atezolizumab, avelumab, durvalumab, nivolumab, pembrolizumab, and cemiplimab, more preferably atezolizumab, avelumab and durvalumab, most preferably atezolizumab. Ipilimumab is an anti-CTLA4 antibody. Nivolumab, pembrolizumab, and cemiplimab are anti-PD1 antibodies. Atezolizumab, avelumab, durvalumab are anti-PD-L1 antibodies.

It will be readily understood that the embodiments outlined above shall apply to the invention as a whole and not be limited to a specific method, unless stated otherwise. It will for example be understood the embodiments for the type of cancer shall be applied to every method, kit or the like disclosed herein. The invention is further illustrated by the following non-limiting examples and figures.

### Examples

### Example 1: Anti-PD1 autoantibody and anti-PD-L1 autoantibody ELISA

Anti-PD1 and Anti-PD-L1 autoantibody levels are measured in serum samples using a sandwich ELISA kit (CellTrend GmbH Luckenwalde, Germany). The microtiter 96-well polystyrene plates were coated with full-length human PD1 (CD279) or full length human PD-L1 (CD274), respectively. To maintain the conformational epitopes of the proteins, 1 mM calcium chloride was added to every buffer. Duplicate samples of a 1:100 serum dilution were incubated at 4°C for 2 hours. After washing steps, plates were incubated for 60 minutes with a 1:20.000 dilution of horseradish-peroxidase-labeled goat anti-human IgG (Jackson, USA) used for detection. In order to obtain a standard curve, plates were incubated with test sera from an autoantibody positive index patients. A monoclonal antibody against PD1 or PD-L1 was used as an positive control. The ELISA was validated according to the FDA's "Guidance for industry: Bioanalytical method validation".

To set a standard for the concentrations of the autoimmuno antibodies, a standard curve was generated. In detail, a serum sample of an autoantibody positive index patientwas diluted (a) 1:200 for standard point 40 Units/ml, (b) 1:400 for standard point 20 Units/ml, (c) 1:800 for standard point 10 Units/ml, (d) 1:1600 for standard point 5 Units/ml, (e) 1:3200 for standard point 2.5 Units/ml and (f) 1:6400 for standard point 1.25 Units/ml. Then the optical density was determined using the kit and method as set out above. Each standard point was performed in duplicates.

### Example 2: Anti-PD1 autoantibody and anti-PD-L1 autoantibody levels in HNSCC patients and healthy donors (HD)

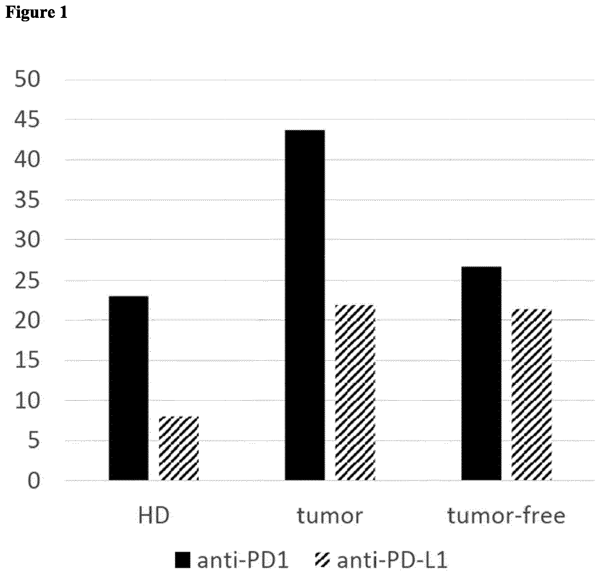

Anti-PD1 and anti-PD-L1 antibody levels in serum samples of healthy donors (HD; n = 4), HNSCC patients with tumor before the beginning of atezolizumab treatment (n = 5) and tumor-free HNSCC patients after atezolizumab treatment (n = 4) were determined using the assay of Example 1. The results are shown in Figure 1.

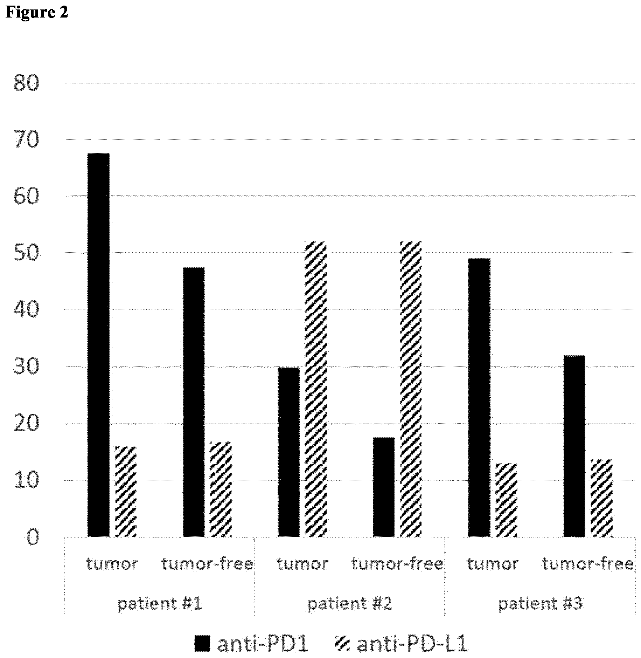

Anti-PD1 and anti-PD-L1 antibody levels in serum samples of three different individual HNSCC patients (#1 to #3) with tumor before the beginning of atezolizumab treatment and after atezolizumab treatment were determined using the assay of Example 1. The results are shown in Figure 2.

## Claims

1. A method for the diagnosis, prognosis, risk assessment, risk stratification, monitoring, therapy guidance and/or therapy control of cancer in a subject comprising the determination of the level of an anti-PD1 auto-antibody and/or an anti-PD-L1 auto-antibody in a blood sample of said subject.

2. The method of claim 1, wherein the level of an anti-PD1 antibody is determined in a sample of said subject and wherein the subject does not receive treatment with an anti-PD1 antibody.

3. The method of claim 1, wherein the level of an anti-PD-L1 antibody is determined in a sample of said subject and wherein the subject does not receive treatment with an anti-PD-L1 antibody.

4. The method according to any of the preceding claims, wherein the level of the anti-PD1 antibody and/or the anti-PD-L1 antibody in the sample is compared to a control level, preferably the control level is derived from a sample of a healthy individual or samples from a group of healthy individuals.

5. The method according to claim 4, wherein a level that is above said control level is indicative for a high tumor load.

6. The method according to any of the preceding claims, wherein the cancer is a solid tumor.

7. The method of claim 6, wherein the cancer is selected from the group consisting of lung cancer, bladder cancer, breast cancer, colorectal cancer, melanoma, renal cell carcinoma (RCC), pancreatic cancer, gastric cancer, liver cancer, gastroesophageal cancer, lymphoma, head and neck squamous cell carcinoma (HNSCC) and ovarian cancer.

8. The method according to any of the preceding claims, wherein the sample is whole blood, serum or plasma.

9. The method according to any one of the preceding claims, wherein the anti-PD1 antibody or the anti-PD-L1 antibody is detected using an immunoassay comprising the steps of
(a) contacting the sample with PD1, PD-L1 or an immunogenic peptide thereof, under conditions allowing for the formation of a complex between said antibody with PD1, PD-L1 or the immunogenic peptide thereof;
(b) detecting the complex.

10. The method of claim 9, wherein PD1, PD-L1 or the immunogenic peptide thereof is immobilized on a surface.

11. The method according to any one of claims 9 or 10, wherein the complex is detected using a secondary antibody against IgG.

12. The method according to claim 11, wherein the secondary antibody is labeled with a detectable marker.

13. The method according to any one of claims 9 to 12, wherein the immunoassay is selected from the group of immunoprecipitation, enzyme immunoassay (EIA), radioimmunoassay (RIA) or fluorescencent immunoassay, a chemiluminescent assay, an agglutination assay, nephelometric assay, turbidimetric assay, a Western blot, a competitive immunoassay, a noncompetitive immunoassay, a homogeneous immunoassay a heterogeneous immunoassay, a bioassay and a reporter-assay such as a Luciferase-Assay, preferably an the immunoassay is an ELISA.

14. The method according to any one of the preceding claims, wherein the subject is a human.

## Patentansprüche

1. Verfahren für die Diagnose, Prognose, Risikoeinschätzung, Risikostratifikation, Kontrolle, Therapieführung und/oderTherapiekontrolle einer Krebserkrankung in einem Individuum, umfassend die Bestimmung des Spiegels eines Anti-PD1-Auto-Antikörpers und/oder eines Anti-PD-L1-Auto-Antikörpers in einer Blutprobe des Individuums.

2. Verfahren nach Anspruch 1, wobei der Spiegel eines Anti-PD1-Antikörpers in einer Probe des Individuums bestimmt wird und wobei das Individuum keine Behandlung mit einem Anti-PD1-Antikörper erhält.

3. Verfahren nach Anspruch 1, wobei der Spiegel eines Anti-PD-L1-Antikörpers in einer Probe des Individuums bestimmt wird und wobei das Individuum keine Behandlung mit einem Anti-PD-L1-Antikörper erhält.

4. Verfahren nach einem der vorangehenden Ansprüche, wobei der Spiegel des Anti-PD1-Antikörpers und/oder des Anti-PD-L1-Antikörpers in der Probe mit einem Kontrollspiegel verglichen wird, bevorzugt stammt der Kontrollspiegel von einer Probe eines gesunden Individuums oder von Proben einer Gruppe gesunder Individuen ab.

5. Verfahren nach Anspruch 4, wobei ein Spiegel, der über dem Kontrollspiegel liegt, ein Hinweis auf eine hohe Tumorbelastung ist.

6. Verfahren nach einem der vorangehenden Ansprüche, wobei die Krebserkrankung ein solider Tumor ist.

7. Verfahren nach Anspruch 6, wobei die Krebserkrankung ausgewählt ist aus der Gruppe bestehend aus Lungenkrebs, Blasenkrebs, Brustkrebs, Darmkrebs, Melanom, Nierenzellkarzinom (renal cell carcinoma, RCC), Bauchspeicheldrüsenkrebs, Magenkrebs, Leberkrebs, Magen- und Speiseröhrenkrebs, Lymphom, Plattenepithelkarzinom des Kopfes und des Halses (head and neck squamous cell carcinoma, HNSCC) und Eierstockkrebs.

8. Verfahren nach einem der vorangehenden Ansprüche, wobei die Probe Vollblut, Serum oder Plasma ist.

9. Verfahren nach einem der vorangehenden Ansprüche, wobei der Anti-PD1-Antikörper oder der Anti-PD-L1-Antikörper unter Verwendung eines Immunoassays nachgewiesen wird, umfassend die Schritte des
(a) Inkontaktbringens der Probe mit PD1, PD-L1 oder einem immunogenen Peptid davon, unter Bedingungen, die die Bildung eines Komplexes zwischen dem Antikörper mit PD1, PD-L1 oder dem immunogenen Peptid davon ermöglichen;
(b) Nachweisens des Komplexes.

10. Verfahren nach Anspruch 9, wobei PD1, PD-L1 oder das immunogene Peptid davon auf einer Oberfläche immobilisiert ist.

11. Verfahren nach einem der Ansprüche 9 oder 10, wobei der Komplex unter Verwendung eines sekundären Antikörpers gegen IgG nachgewiesen wird.

12. Verfahren nach Anspruch 11, wobei der sekundäre Antikörper mit einem nachweisbaren Marker markiert ist.

13. Verfahren nach einem der Ansprüche 9 bis 12, wobei der Immunoassay ausgewählt ist aus der Gruppe bestehend aus Immunpräzipitation, Enzym-Immunoassay (EIA), RadioImmunoassay (RIA) oder Fluoreszenz-Immunoassay, einem Chemilumineszenz-Assay, einem Agglutinations-Assay, nephelometrischer Assay, turbidimetrischer Assay, einem Western Blot, einem kompetitiven Immunoassay, einem nicht-kompetitiven Immunoassay, einem homogenen Immunoassay, einem heterogenen Immunoassay, einem Bioassay und einem Reporter-Assay wie einem Luciferase-Assay, bevorzugt ist der Immunoassay ein ELISA.

14. Verfahren nach einem der vorangehenden Ansprüche, wobei das Individuum ein Mensch ist.

## Revendications

1. Méthode pour le diagnostic, le pronostic, l'évaluation du risque, la stratification du risque, la surveillance, le guidage thérapeutique et/ou le contrôle thérapeutique d'un cancer dans un sujet comprenant la détermination du niveau d'un auto-anticorps anti-PD1 et/ou d'un auto-anticorps anti-PD-L1 dans un échantillon de sang dudit sujet.

2. Méthode selon la revendication 1, dans laquelle le niveau d'un anticorps anti-PD1 est déterminé dans un échantillon dudit sujet et dans laquelle le sujet ne reçoit pas de traitement avec un anticorps anti-PD1.

3. Méthode selon la revendication 1, dans laquelle le niveau d'un anticorps anti-PD-L1 est déterminé dans un échantillon dudit sujet et dans laquelle le sujet ne reçoit pas le traitement avec un anticorps anti-PD-L1.

4. Méthode selon l'une quelconque des revendications précédentes, dans laquelle le niveau de l'anticorps anti-PD1 et/ou de l'anticorps anti-PD-L1 dans l'échantillon est comparé à un niveau de contrôle, de préférence le niveau de contrôle est dérivé d'un échantillon d'un individu en bonne santé ou d'échantillons provenant d'un groupe d'individus en bonne santé.

5. Méthode selon la revendication 4, dans laquelle un niveau qui est au-dessus dudit niveau de contrôle indique une charge tumorale élevée.

6. Méthode selon l'une quelconque des revendications précédentes, dans laquelle le cancer est une tumeur solide.

7. Méthode selon la revendication 6, dans laquelle le cancer est choisi dans le groupe constitué du cancer du poumon, cancer de la vessie, cancer du sein, cancer colorectal, mélanome, carcinome à cellules rénales (CCR), cancer du pancréas, cancer gastrique, cancer du foie, cancer gastro-oesophagien, lymphome, carcinome à cellules squameuses de la tête et du cou (HNSCC) et cancer ovarien.

8. Méthode selon l'une quelconque des revendications précédentes, dans laquelle l'échantillon est du sang total, du sérum ou du plasma.

9. Méthode selon l'une quelconque des revendications précédentes, dans laquelle l'anticorps anti-PD1 ou l'anticorps anti-PD-L1 est détecté au moyen d'un immunoessai comprenant les étapes de
(a) mise en contact de l'échantillon avec PD1, PD-L1 ou un peptide immunogène de celui-ci, dans des conditions permettant la formation d'un complexe entre ledit anticorps avec PD1, PD-L1 ou le peptide immunogène de celui-ci ;
(b) détection du complexe.

10. Méthode selon la revendication 9, dans laquelle PD1, PD-L1 ou le peptide immunogène de celui-ci est immobilisé sur une surface.

11. Méthode selon l'une quelconque des revendications 9 ou 10, dans laquelle le complexe est détecté au moyen d'un anticorps secondaire contre lgG.

12. Méthode selon la revendication 11, dans laquelle l'anticorps secondaire est marqué avec un marqueur détectable.

13. Méthode selon l'une quelconque des revendications 9 à 12, dans laquelle l'immunoessai est choisi dans le groupe de l'immunoprécipitation, l'essai immunoenzymatique (EIA), le radioimmunoessai (RIA) ou l'immunoessai fluorescent, un essai chimiluminescent, un essai d'agglutination, un essai néphélométrique, un essai turbidimétrique, un buvardage Western, un immunoessai compétitif, un immunoessai non compétitif, un immunoessai homogène, un immunoessai hétérogène, un essai biologique et un essai rapporteur tel qu'un essai de luciférase, de préférence un l'immunoessai est un ELISA.

14. Méthode selon l'une quelconque des revendications précédentes, dans laquelle le sujet est un humain.
